# EUROPÄISCHE PATENTSCHRIFT

(11) **EP 1 148 042 B1**
(45) Veröffentlichungstag und Bekanntmachung des Hinweises auf die Patenterteilung: **06.07.2005**
(21) Anmeldenummer: 01106810.3
(22) Anmeldetag: 19.03.2001
(51) Int. Cl.: C07C 51/08, C07C 51/43, C07C 59/56, C12P 41/00, C12P 13/00

(54) **Verfahren zur Herstellung von optisch und chemisch hochreinen (R)- oder (S)-Hydroxycarbonsäuren**
Method for producing optically and chemically pure (R)- and (S)-hydroxycarboxylic acids
Procédé de préparation de l'acide (R)- et (S)- hydroxycarboxylique optiquement et chimiquement pur

(30) Priorität: 17.04.2000 AT 6702000
(43) Veröffentlichungstag der Anmeldung: 24.10.2001
(73) Patentinhaber: DSM Fine Chemicals Austria Nfg GmbH & Co KG, 4021 Linz (AT)
(72) Erfinder: Pöchlauer, Peter, 4040 Linz (AT); Mayrhofer, Herbert, 4210 Engerwitzdorf (AT)
(74) Vertreter: Lindinger, Ingrid

(56) Entgegenhaltungen:
- EFFENBERGER, F.: "Synthesis and reactions of optically active cyanohydrins" ANGEWANDTE CHEMIE, INTERNATIONAL EDITION ENGLISCH, Bd. 33, 1994, Seiten 1555-1564, XP002222654
- DATABASE CA [Online] CHEMICAL ABSTRACTS SERVICE, COLUMBUS, OHIO, US; T., ADRIAN CAPARACHIN: "Synthesis of dl- mandelic acid" retrieved from STN Database accession no. 48:7147 XP002222656 & ANALES FAC. FARM. Y BIOQUIM., UNIV. NACL. MAYOR SAN MARCOS (LIMA, PERU) (1950), 1, 615-21,
- DATABASE CA [Online] CHEMICAL ABSTRACTS SERVICE, COLUMBUS, OHIO, US; MITSUI, TETSUO ET AL: "Synthesis of substances having the properties of plantgrowth hormones. VI The growth-promoting activity for plants of substances which have one or more carboxylic groups attached directly to the ring system" retrieved from STN Database accession no. 47:54834 XP002222657 & J. AGR. CHEM. SOC. JAPAN (1952), VOLUME DATE 1951-1952, 25, 63-6,
- EFFENBERGER, F. ET AL.: "Enzyme-catalyzed synthesis of (R)-ketone-cyanohydrins and their hydrolysis to (R)-alpha-hydroxy-alpha-methyl-carboxylic acids" TETRAHEDRON LETTERS, Bd. 32, Nr. 23, 1991, Seiten 2605-2608, XP002222653
- GREGORY, R. J. H.: "Cyanohydrins in nature and the laboratory: Biology, preparations, and synthetic applications" CHEMICAL REVIEWS, Bd. 99, 1999, Seiten 3649-3682, XP002222655
- DATABASE WPI Section Ch, Week 198918 Derwent Publications Ltd., London, GB; Class B05, AN 1989-134294 XP002222658 & JP 01 079134 A (KANEGAFUCHI CHEM KK), 24. März 1989 (1989-03-24)

## Beschreibung

Optisch aktive α-Hydroxycarbonsäuren finden beispielsweise als Zusatzstoffe zu Futtermitteln oder zur Gewinnung pharmazeutischer Wirkstoffe, Vitamine und Flüssigkristalle Verwendung.

Diese optisch aktiven α-Hydroxycarbonsäuren lassen sich weiters beispielsweise nach Effenberger et al., Angew. Chem. 95 (1983) Nr.1, Seite 50, vorteilhaft in anderweitig nur sehr schwer herzustellende N-substituierte optisch aktiven α-Aminosäuren überführen.

Chirale α-Hydroxycarbonsäuren sind heutzutage chemisch, fermentativ oder enzymatisch zugänglich. Die säurekatalysierte Hydrolyse von Cyanhydrinen stellt dabei eine wichtige Herstellungsvariante dar. So können beispielsweise racemische Cyanhydrine unter Zusatz geeigneter Mikroorganismen zu den gewünschten chiralen α-Hydroxycarbonsäuren hydrolysiert werden.

Die Herstellung racemischer Hydroxycarbonsäuren durch saure Hydrolyse von racemischen Cyanhydrinen ist beispielsweise aus Database 48: 7147; XP-002222656 oder aus Database 47: 54834; XP-002222657 bekannt.

Es ist auch bekannt, beispielsweise aus Angew. Chem. 1994, 106, Seite 1615f., Angew. Chemie, Intemational Edition, 1994, 33, Seiten 1555-1564 oder aus Tetrahedrn Letters, 32, 2605-2608 (1991), dass optisch aktive Cyanhydrine, die beispielsweise leicht mittels enzymkatalysierter Synthese erhältlich sind, ohne Racemisierung zu den korrespondierenden chiralen α-Hydroxycarbonsäuren hydrolysiert werden können. Die optische Reinheit der so hergestellten chiralen α-Hydroxycarbonsäure entspricht dabei der optischen Reinheit des eingesetzten chiralen Cyanhydrins, auch wenn dieses in situ durch enzymkatalysierte Addition von einem Cyanidgruppendonor an einen entsprechenden Aldehyd oder ein Keton erhalten und ohne Isolierung bzw. Aufreinigung weiterverarbeitet wird. Die Aufarbeitung derart hergestellter α-Hydroxycarbonsäuren erfolgte bisher mittels Extraktion. Diese optische Reinheit kann jedoch für gewisse Anwendungsbereiche der α-Hydroxycarbonsäuren, beispielsweise bei Anwendung im Pharmabereich, zu gering sein, sodass die Notwendigkeit bestand bzw. es Aufgabe der Erfindung war, eine Möglichkeit zu finden, gleichzeitig die chemische und die optische Reinheit der α-Hydroxycarbonsäuren auf einfachem Weg, ohne großen Ausbeuteverlust, zu steigern.
Unerwarteterweise konnte diese Aufgabe durch einen Kristallisationsschritt in einem aromatischen Kohlenwasserstoff gelöst werden.

Gegenstand der Erfindung ist demnach ein Verfahren zur Herstellung von chemisch und optisch hochreinen *(R)-* und *(S)-α*-Hydroxycarbonsäuren, das dadurch gekennzeichnet ist, dass verunreinigte *(R)-* und *(S)-*α-Hydroxycarbonsäuren, hergestellt durch saure Hydrolyse der durch enzymkatalysierten Addition eines Cyanidgruppendonors an die entsprechenden Aldehyde oder Ketone erhaltenen *(R)*- und *(S)*- Cyanhydrinen, in einem aromatischen Kohlenwasserstoff, gegebenenfalls in Anwesenheit eines Cosolvens, umkristallisiert werden und chemisch und optisch hochreine *(R)-* und *(S)*-α-Hydroxycarbonsäuren erhalten werden.

Bei dem erfindungsgemäßen Verfahren werden verunreinigte *(R)-* und *(S)*-α-Hydroxycarbonsäuren in hochreine *(R)-* und *(S)*-α-Hydroxycarbonsäuren mit einer optischen Reinheit von über 98%ee überführt.

Als Ausgangsverbindungen dienen *(R)-* und *(S)*-α-Hydroxycarbonsäuren, die durch saure Hydrolyse der korrespondierenden *(R)*- und *(S)*- Cyanhydrine erhalten werden, die wiederum durch enzymatische Addition eines Cyanidgruppendonors an die entsprechenden Aldehyde oder Ketone hergestellt werden.

Die enzymatische Addition eines Cyanidgruppendonors an die entsprechenden Aldehyde oder Ketone kann dabei analog dem Stand der Technik, beispielsweise analog EP 0 951 561, EP 0 927 766, EP 0 632 130, EP 0547 655, EP 0 326 063 erfolgen.
Als Ausgangsverbindungen eignen sich die im Stand der Technik zitierten Aldehyde und Ketone.
Beispiele für geeignete Aldehyde sind dabei aliphatische, aromatische oder heteroaromatische Aldehyde. Unter aliphatischen Aldehyden sind dabei gesättigte oder ungesättigte aliphatische, geradkettige, verzweigte oder cyclische Aldehyde zu verstehen. Bevorzugte aliphatische Aldehyde sind geradkettige Aldehyde mit insbesondere 2 bis 18 C-Atomen, bevorzugt von 2 bis 12, die gesättigt oder ein- oder mehrfach ungesättigt sind. Der Aldehyd kann dabei sowohl C-C-Doppelbindungen als auch C-C-Dreifachbindungen aufweisen. Der Aldehyd kann unsubstituiert oder ein- oder mehrfach durch unter den Reaktionsbedingungen inerte Gruppen, beispielsweise durch gegebenenfalls substituierte Aryl- oder Heteroarylgruppen, wie Phenyl- oder Indolylgruppen, durch C₁-C₆-Alkyl, gegebenenfalls substituierte Cycloalkylgruppen, die ein oder mehrere Heteroatome aus der Gruppe O, S, P, oder N aufweisen können, Halogen-, Ether-, Alkohol-, Acyl-, Carbonsäure-, Carbonsäureester-, Nitro- oder Azidogruppen substituiert sein.
Beispiele für aromatische oder heteroaromatische Aldehyde sind Benzaldezyd bzw. verschieden substituierte Benzaldehyde wie etwa 2-Chlorbenzaldehyd, 3,4-Difluorbenzaldehyd, 3-Phenoxybenzaldehyd, 4-Fluor-3-phenoxybenzaldehyd, weiters Furfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd oder Pyridinaldehyde.
Beispiele für Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder ein- oder mehrfach durch unter Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Ketone sind Acetophenon oder Indolylaceton.

Das entsprechende *(R)-* und *(S)-* Cyanhydrin wird sodann, beispielsweise nach Extraktion oder nach Abfiltrieren des Enzyms und Abdestillieren des Lösungsmittels, ohne weitere Reinigung analog dem Stand der Technik, beispielsweise wie in Angew. Chem. 1994, 106, S 1615 oder in Tetrahedron Letters, Vol. 31, No. 9, pp1249-1252, 1990 beschrieben, mit konzentrierter Salzsäure hydrolysiert.

Die so erhaltenen rohen *(R)-* und *(S)*-α-Hydroxycarbonsäuren, die in etwa die gleiche optische Reinheit wie die korrespondierenden *(R)-* und *(S)-* Cyanhydrine aufweisen, werden sodann durch Extraktion aus dem Reaktionsgemisch isoliert und erfindungsgemäß in einem aromatischen Kohlenwasserstoff, gegebenenfalls in Anwesenheit eines Cosolvens, umkristallisiert.
Geeignete aromatische Kohlenwasserstoffe sind dabei Toluol, Xylole, Benzol, oder substituierte Benzole, wie etwa Ethylbenzol, Isopropylbenzol oder Chlorbenzole. Bevorzugt werden Toluol und Xylol eingesetzt.
Als Cosolvens eignen sich solche Lösungsmittel, die die Löslichkeit der Hydroxycarbonsäure in der organischen Phase erhöhen und die destillativ leicht abtrennbar sind. Beispiele dafür sind gegebenenfalls cyclische Ether, wie etwa Tetrahydrofuran, Methyl-tert.Butylether, Dimethoxyethan oder Ketone, wie etwa Methyl-iso-Butylketon.
Die Menge an Cosolvens beträgt dabei 5 bis 50 Vol%, bevorzugt 10 bis 30 Vol%, bezogen auf die Gesamtmenge an Lösungsmittel.
Die zu reinigende α-Hydroxycarbonsäure wird in dem entsprechenden Lösungsmittel bzw. Lösungsmittelgemisch unter Erwärmen gelöst, worauf nach Trennung der Phasen, von der organischen Phase zur Entfernung des Wassers bevorzugt ein Teil des Lösungsmittels bzw. des Lösungsmittelgemisches abdestilliert wird und anschließend langsam auf 15-50°C abgekühlt wird. Nach einer Stehzeit von wenigen Minuten bis zu mehreren Stunden (5 Minuten bis 20 Stunden, längere Stehzeiten sind nötigenfalls auch möglich) wird das auskristallisierte Produkt abfiltriert, das Kristallisat mit dem gleichen Lösungsmittel bevorzugt 1 bis 5 mal nachgewaschen und getrocknet.
Dabei werden hochreine *(R)-* und *(S)-α*-Hydroxycarbonsäuren mit einer optischen Reinheit von über 98%ee erhalten. Das erfindungsgemäße Verfahren ermöglicht es dabei, über 90% der Verunreinigungen in einem Arbeitsschritt abzutrennen.

Bevorzugt wird das erfindungsgemäße Verfahren für aromatische *(R)-* und *(S)*-α-Hydroxycarbonsäuren der Formel Ar-(CH₂)ₙCH(OH)CO₂H eingesetzt. In der Formel Ar-(CH₂)ₙCH-R-CO₂H bedeuten n 0, oder eine ganze Zahl von 1 bis 5 und Ar einen unsubstituierten oder ein oder mehrfach substituierten Aryl- oder Heteroarylrest, wie etwa Phenyl, Benzyl, Naphthyl, Pyridyl, Furyl, wobei als Substituenten beispielsweise OH, C₁-C₄-Alkyl oder -Alkoxy, Thioalkyl, Halogen, gegebenenfalls substituiertes Phenyl oder Phenoxy, Amino oder Nitro in Frage kommen.
Besonders bevorzugt ist n = 0, 1 oder 2 und Ar ein Arylrest, insbesondere Phenyl, der unsubstituiert oder bevorzugt durch C₁-C₄-Alkyl oder -Alkoxy, OH, Cl, Br, Phenyl, Phenoxy, Fluorphenoxy substituiert sein kann.
In einer ganz bevorzugten Ausführungsform wird das erfindungsgemäße Verfahren zur Reinigung von *(R)*-2-Chlormandelsäure angewandt.
Dabei wird zuerst optisch aktives *(R)*-2-Chlormandelonitril durch enzymkatalysierte Anlagerung von Blausäure an 2-Chlorbenzaldehyd mittels R-Oxynitrilase hergestellt, das Cyanhydrin durch Extraktion aus dem Reaktionsgemisch isoliert und Lösungsmittel abdestilliert. Das Cyanhydrin weist dabei einen Enantiomerenüberschuss von etwa 89-92%ee (ca. 95% R und ca. 5% S) auf.
Ohne weitere Aufreinigung wird das Cyanhydrin in konzentrierter Salzsäure bei üblichen Bedingungen, wie etwa 60°C und einer Dauer von 24 Stunden, hydrolysiert und anschließend die entsprechende Hydroxycarbonsäure extrahiert. Bei der Hydrolyse kommt es, wie aus dem Stand der Technik bekannt, zu keiner Racemisierung, sodass das Rohprodukt ebenfalls eine optische Reinheit von etwa 89-92%ee (ca. 95% R und ca. 5% S) aufweist. Durch das erfindungsgemäße Kristallisieren in einem aromatischen Kohlenwasserstoff, gegebenenfalls in Anwesenheit eines Cosolvens, wird neben der chemischen Reinheit auch die optische Reinheit in äußerst einfacher und wirtschaftlicher Weise verbessert und es wird optisch hochreine *(R)*-2-Chlormandelsäure mit einer optischen Reinheit von über 98% ee mit über 99% R-Form und weniger als 1 % S-Form erhalten.

Weiters ist es möglich den erfindungsgemäßen Kristallisationsschritt direkt an den Hydrolyseschritt zu koppeln, sodass das bisher übliche Extrahieren mit im Stand der Technik als Extraktionsmitteln verwendeten Ethern, wie Methyl-tert.Butylether oder Diethylether, der α-Hydroxycarbonsäuren aus dem Reaktionsgemisch entfällt.
Erfindungsgemäß wird dabei der nach der gemäß dem Stand der Technik durchgeführten Hydrolyse des entsprechenden Cyanhydrins in wässrig saurer Lösung erhaltenen Hydrolyselösung bei Hydrolysetemperatur (30 bis 110°C) ein aromatischer Kohlenwasserstoff, gegebenenfalls in Kombination mit einem Cosolvens, zugesetzt.
Geeignete aromatische Kohlenwasserstoffe sind dabei wiederum Toluol, Xylole, Benzol, oder substituierte Benzole, wie etwa Ethylbenzol, Isopropylbenzol oder Chlorbenzole. Bevorzugt werden Toluol und Xylol eingesetzt. Als Cosolvens eignen sich wiederum die bereits angeführten Lösungsmittel.
Nach 1 bis Smaligen, bevorzugt 1 bis 3maligen Extrahieren mit dem entsprechenden aromatischen Kohlenwasserstoff bzw. Lösungsmittelgemisch werden die wässrigen Phasen verworfen, worauf von den vereinigten organischen Phasen zur Entfernung des Wassers bevorzugt ein Teil des Lösungsmittels bzw. des Lösungsmittelgemisches abdestilliert und anschließend die verbleibende organische Phase abgekühlt wird, wodurch eine chemisch und optisch hochreine α-Hydroxycarbonsäure auskristallisiert, die eine optische Reinheit von über 98%ee aufweist. Das Kristallisat wird sodann abfiltriert, gegebenenfalls nachgewaschen und von Resten an Lösungsmittel befreit, sowie getrocknet.

Die erfindungsgemäße direkt an den Hydrolyseschritt angekoppelte Kristallisation, durch die ein zusätzlicher Isolierungsschritt entfällt, eignet sich ebenfalls für alle *(R)*- und *(S)*- Cyanhydrine, die durch enzymatische Addition eines Cyanidgruppendonors an die entsprechenden Aldehyde oder Ketone hergestellt werden.

Ein weiterer Gegenstand der vorliegenden Erfindung ist demnach ein Verfahren zur Herstellung von chemisch und optisch hochreinen *(R)-* und *(S)*-α-Hydroxycarbonsäuren, das dadurch gekennzeichnet ist, dass die durch saure Hydrolyse der durch enzymkatalysierte Addition eines Cyanidgruppendonors an die entsprechenden Aldehyde oder Ketone hergestellten *(R)*- und *(S)*- Cyanhydrine erhaltene Hydrolyselösung direkt mit einem aromatischen Kohlenwasserstoff, gegebenenfalls in Kombination mit einem Cosolvens, versetzt wird, anschließend bei Hydrolysetemperatur extrahiert wird, worauf nach Abkühlen der organischen Phase die entsprechenden chemisch und optisch hochreinen *(R)-* und *(S)*-α-Hydroxycarbonsäuren mit einer optischen Reinheit von über 98%ee auskristallisieren.

Bevorzugt wird das erfindungsgemäße Verfahren wiederum für aromatische *(R)-* und *(S)*-α-Hydroxycarbonsäuren der Formel Ar-(CH₂)ₙCH(OH)CO₂H eingesetzt. In der Formel Ar-(CH₂)ₙCH-R-CO₂H bedeuten n=0, oder eine ganze Zahl von 1 bis 5 und Ar einen unsubstituierten oder ein oder mehrfach substituierten Aryl- oder Heteroarylrest, wie etwa Phenyl, Benzyl, Naphthyl, Pyridyl, Furyl, wobei als Substituenten beispielsweise OH, C₁-C₄-Alkyl oder -Alkoxy, Thioalkyl, Halogen, gegebenenfalls substituiertes Phenyl oder Phenoxy, Amino oder Nitro in Frage kommen.
Besonders bevorzugt ist n = 0, 1 oder 2 und Ar ein Arylrest, insbesondere Phenyl, der unsubstituiert oder bevorzugt durch C₁-C₄-Alkyl oder -Alkoxy, OH, Cl, Br, Phenyl, Phenoxy, Fluorphenoxy substituiert sein kann.
In einer ganz bevorzugten Ausführungsform wird das erfindungsgemäße Verfahren zur Herstellung von chemisch und optisch hochreinen *(R)*-2-Chlormandelsäure angewandt.
Dabei wird zuerst optisch aktives *(R)*-2-Chlormandelonitril durch enzymkatalysierte Anlagerung von Blausäure an 2-Chlorbenzaldehyd mittels R-Oxynitrilase hergestellt, das Cyanhydrin durch Extraktion aus dem Reaktionsgemisch isoliert und Lösungsmittel abdestilliert. Das Cyanhydrin weist dabei einen Enantiomerenüberschuss von etwa 89-92%ee (ca. 95% R und ca. 5% S) auf.
Ohne weitere Aufreinigung wird das Cyanhydrin in konzentrierter Salzsäure bei üblichen Bedingungen, wie etwa 60°C und einer Dauer von 24 Stunden, hydrolysiert und anschließend die so erhaltene Hydrolyselösung direkt mit einem aromatischen Kohlenwasserstoff, anstelle der bisher üblichen Ether, gegebenenfalls in Kombination mit einem Cosolvens, versetzt und 1 bis 5 Mal extrahiert. Die wässrigen Phasen werden verworfen; aus den vereinigten organischen Phasen kristallisiert durch Abkühlen derselben die chemisch und optisch hochreine *(R)*-2-Chlormandelsäure aus. Durch die erfindungsgemäße Koppelung der Kristallisation an die Hydrolyse wird neben der chemischen Reinheit auch die optische Reinheit in äußerst einfacher und wirtschaftlicher Weise verbessert und es wird chemisch und optisch hochreine *(R)*-2-Chlormandelsäure mit einer optischen Reinheit von über 98% ee mit über 99% R-Form und weniger als 1 % S-Form erhalten.

### Beispiel 1:

453,5 g R-2-Chlormandelonitril wurden in 925 ml Salzsäure konz. 24 Stunden lang bei 63 °C gerührt. Nach dem Abkühlen wurde mit 812 ml Wasser und 1255 ml tert.-Butylmethylether versetzt und heftig gerührt. Die Phasen wurden getrennt und die wäßrige Phase anschließend nochmals mit 1255 ml tert.-Butylmethylether extrahiert. Die vereinten organischen Phasen wurden danach mit 185 ml Wasser extrahiert. Von der dunkelbraunen organischen Phase wurde das Lösungsmittel im Vakuum abdestilliert.
Die Auswaage betrug 497,3 g und die Enantiomerenreinheit des braunen Rohproduktes wurde mittels GC Analyse bestimmt (91,5 % ee = 95,7 % R und 4,3 % S).
Das Rohprodukt wurde durch Erwärmen auf 100 °C in 2780 ml Xylol gelöst. Anschließend wurde langsam auf 21 °C abgekühlt, wobei die Kristallisation bei 88 °C einsetzte. Nach einer Stehzeit von 16 Stunden wurde abfiltriert und das Kristallisat am Filter mit 200 ml Xylol gewaschen.
Anschließend wurde das Produkt noch einmal mit 880 ml und zweimal mit 550 ml Xylol gewaschen. Nach dem Trockensaugen wurden die Reste an Lösungsmittel bei 60 °C und 10 mbar entfernt.

Es wurden 405,9 g weiße, kristalline R-2-Chlormandelsäure mit 98,1 % ee (99,05 % R und 0,95 % S-Enantiomer) gewonnen.

Die Mutterlauge wurde einrotiert, wobei eine dunkelbraune zähe Masse als Rückstand verblieb. Die darin enthaltene 2-Chlormandelsäure hatte ein Enantiomerenverhältnis von 51,9 % R zu 48,1 % S.

### Beispiel 2:

16,2 g R-2-Chlormandelonitril wurden in 32 ml Salzsäure konz. 24 Stunden lang bei 63 °C gerührt. Es wurde auf 76°C erwärmt und mit 100 ml Xylol versetzt und heftig gerührt. Die Phasen wurden getrennt und die wäßrige Phase anschließend nochmals mit 50 ml Xylol extrahiert.
Von der dunkelbraunen organischen Phase, welche R-2-Chlormandelsäure mit 91,4 % ee enthielt, wurde ein Teil des Lösungsmittels zur Entfernung von Wasser im Vakuum abdestilliert. Anschließend wurde langsam auf 21 °C abgekühlt, wobei die Kristallisation einsetzte. Nach einer Stehzeit von 16 Stunden wurde abfiltriert und das Kristallisat am Filter mit 8 ml Xylol gewaschen.
Anschließend wurde das Produkt noch einmal mit 31 ml und zweimal mit 20 ml Xylol gewaschen. Nach dem Trockensaugen wurden die Reste an Lösungsmittel bei 60 °C und 10 mbar entfernt.
Es wurde hochreine R-2-Chlormandelsäure mit 99,2 % ee erhalten.

### Beispiel 3:

42,3 ml einer durch Hydrolyse erhaltenen Lösung von 16,2 g R-2-Chlormandelonitril in 32 ml Salzsäure konz. wurden auf 5 ml Portionen aufgeteilt, bei 80 °C mit 15 ml an in der Tabelle angegebenen Lösungsmitteln bzw. Lösungsmittelgemischen extrahiert und die angegebenen Gehalte an R-2-Chlormandelsäure, mit einer Enantiomerenreinheit von 91,4 % ee, in der org. Phase erhalten.

| Extraktionsmittel | Mschungsverhältnis | Gehalt an Produkt in der organischen Phase |
|---|---|---|
| Xylol/Tetrahydrofuran | 80/20 | 1,79g |
| Xylol/Methylisobutylketon | 80/20 | 1,60g |
| Xylol/Dimethoxyethan | 80/20 | 1,42g |
| Xylol | - | 0,63g |
| Toluol | - | 0,79g |

Anschließend wurde Wasser mit einem Teil der organischen Phase abdestilliert und durch Abkühlen der organischen Phase hochreine R-2-Chlormandelsäure mit einer Enantiomerenreinheit von über 98 % ee erhalten. Zum Teil wurden Enantiomerenreinheiten von über 99% ee erhalten; so ergab die Verwendung von Xylol eine Enantiomerenreinheit von 99,2% ee und die Verwendung von Xylol/Tetrahydrofuran eine Enantiomerenreinheit von 99,9% ee.

### Beispiel 4:

13,1 g R-2-Chlormandelonitril (Enantiomerenreinheit: 91,2 % ee) wurden in 30 ml Salzsäure konz. 24 Stunden lang bei 63 °C gerührt. Es wurde auf 80°C erwärmt und mit 100 ml einer Mischung von Xylol und Tetrahydrofuran 80 : 20 heftig gerührt. Die Phasen wurden getrennt und die wässrige Phase anschließend nochmals mit 50 ml Lösungsmittelgemisch extrahiert. Von der dunkelbraunen organischen Phase wurde ein Teil des Lösungsmittelgemisches zur Entfernung von Wasser im Vakuum abdestilliert. Im Destillationssumpf verblieben ca. 80 ml Xylol. Der Destillationssumpf wurde heiß filtriert und anschließend langsam auf Raumtemperatur abgekühlt, wobei die Hydroxycarbonsäure kristallisierte. Nach einer Stehzeit von 16 Stunden wurde abfiltriert und das Kristallisat einmal mit 25 ml Xylol und zweimal mit 16 ml Xylol gewaschen. Nach dem Trockensaugen wurden die Reste an Lösungsmittel bei 60 °C und 10 mbar entfernt.
Es wurden 8,4 g hochreine R-2-Chlormandelsäure mit 99,9 % ee erhalten.

## Patentansprüche

1. Verfahren zur Herstellung von optisch und chemisch hochreinen *(R)-* und *(S)*-α-Hydroxycarbonsäuren, **dadurch gekennzeichnet, dass** verunreinigte *(R)-* und *(S)*-α-Hydroxycarbonsäuren, hergestellt durch saure Hydrolyse der durch enzymkatalysierten Addition eines Cyanidgruppendonors an die entsprechenden Aldehyde oder Ketone erhaltenen *(R)*- und *(S)* - Cyanhydrinen, in einem aromatischen Kohlenwasserstoff, gegebenenfalls in Anwesenheit eines Cosolvens, umkristallisiert werden und optisch und chemisch hochreine *(R)-* und *(S)*-α-Hydroxycarbonsäuren erhalten werden.

2. Verfahren nach Anspruch 1, **dadurch gekennzeichnet, dass** die verunreinigten *(R)-* und *(S)*-α-Hydroxycarbonsäuren durch saure Hydrolyse der durch enzymkatalysierte Addition eines Cyanidgruppendonors an die entsprechenden gegebenenfalls substituierten aliphatischen, aromatischen oder heteroaromatischen Aldehyde oder Ketone erhaltenen *(R)-* und *(S)*- Cyanhydrinen hergestellt werden.

3. Verfahren nach Anspruch 1, **dadurch gekennzeichnet, dass** verunreinigte, aromatische *(R)-* und *(S)*-α-Hydroxycarbonsäuren der Formel Ar-(CH₂)ₙCH(OH)CO₂H, in der n 0 oder eine ganze Zahl von 1 bis 5 bedeutet und Ar ein unsubstituierter oder durch OH, C₁-C₄-Alkyl oder -Alkoxy, Thioalkyl, Halogen, gegebenenfalls substituiertes Phenyl oder Phenoxy, Amino oder Nitro ein oder mehrfach substituierter Aryl- oder Heteroarylrest sein kann, eingesetzt werden.

4. Verfahren nach Anspruch 1, **dadurch gekennzeichnet, dass** *(R)*-2-Chlormandelsäure eingesetzt wird.

5. Verfahren nach Anspruch 1, **dadurch gekennzeichnet, dass** die zu reinigende α-Hydroxycarbonsäure in dem entsprechenden Lösungsmittel unter Erwärmen gelöst wird, anschließend langsam auf 15-50°C abgekühlt wird und nach einer Stehzeit von wenigen Minuten bis zu mehreren Stunden das auskristallisierte Produkt abfiltriert, das Kristallisat mit dem gleichen Lösungsmittel nachgewaschen und getrocknet wird.

6. Verfahren zur Herstellung von chemisch und optisch hochreinen *(R)-* und *(S)*-α-Hydroxycarbonsäuren, **dadurch gekennzeichnet, dass** die durch saure Hydrolyse der durch enzymkatalysierte Addition eines Cyanidgruppendonors an die entsprechenden Aldehyde oder Ketone hergestellten *(R)*- und *(S)*- Cyanhydrine erhaltene Hydrolyselösung direkt mit einem aromatischen Kohlenwasserstoff, gegebenenfalls in Kombination mit einem Cosolvens, versetzt wird, anschließend bei Hydrolysetemperatur extrahiert wird, worauf nach Abkühlen der organischen Phase die entsprechenden chemisch und optisch hochreinen *(R)-* und *(S)*-α-Hydroxycarbonsäuren auskristallisieren.

7. Verfahren nach Anspruch 6, **dadurch gekennzeichnet, dass** chemisch und optisch hochreine aromatische *(R)-* und *(S)*-α-Hydroxycarbonsäuren der Formel Ar-(CH₂)ₙCH(OH)CO₂H, in der n 0 oder eine ganze Zahl von 1 bis 5 bedeutet und Ar ein unsubstituierter oder durch OH, C₁-C₄-Alkyl oder -Alkoxy, Thioalkyl, Halogen, gegebenenfalls substituiertes Phenyl oder Phenoxy, Amino oder Nitro ein oder mehrfach substituierter Aryl- oder Heteroarylrest sein kann, hergestellt werden.

8. Verfahren nach Anspruch 1 oder 6, **dadurch gekennzeichnet, dass** Toluol, Xylole, Benzol, Ethylbenzol, Isopropylbenzol oder Chlorbenzole als aromatische Kohlenwasserstoffe eingesetzt werden.

9. Verfahren nach Anspruch 1 oder 6, **dadurch gekennzeichnet, dass** als Cosolvens ein Lösungsmittel, das die Löslichkeit der Hydroxycarbonsäure in der organischen Phase erhöht und das destillativ leicht abtrennbar ist, in einer Menge von 5 bis 50 Vol% eingesetzt wird.

## Claims

1. Process for the preparation of optically and chemically highly pure *(R)*- and *(S)*-α-hydroxycarboxylic acids, **characterized in that** it comprises recrystallizing impure *(R)-* and *(S)*-α-hydroxycarboxylic acids, prepared by acidic hydrolysis of the *(R)-* and *(S)*-cyanohydrins obtained by enzyme-catalysed addition of a cyanide group donor to the corresponding aldehydes or ketones, in an aromatic hydrocarbon, optionally in the presence of a cosolvent, and obtaining optically and chemically highly pure *(R)-* and *(S)*-α-hydroxycarboxylic acids.

2. Process according to Claim 1, **characterized in that** the impure *(R)-* and *(S)*-α-hydroxycarboxylic acids are prepared by acidic hydrolysis of the (*R*)- and *(S)*-cyanohydrins obtained by enzyme-catalysed addition of a cyanide group donor to the corresponding optionally substituted aliphatic, aromatic or heteroaromatic aldehydes or ketones.

3. Process according to Claim 1, **characterized in that** impure, aromatic *(R)-* and *(S)*-α-hydroxycarboxylic acids of the formula Ar-(CH₂)ₙCH(OH)CO₂H in which n is 0 or an integer from 1 to 5 and Ar is an aryl or heteroaryl radical which is unsubstituted or mono- or polysubstituted by OH, C₁-C₄-alkyl or -alkoxy, thioalkyl, halogen, optionally substituted phenyl or phenoxy, amino or nitro, are employed.

4. Process according to Claim 1, **characterized in that** *(R)*-2-chloromandelic acid is employed.

5. Process according to Claim 1, **characterized in that** the α-hydroxycarboxylic acid to be purified is dissolved in the appropriate solvent with warming, then the solution is slowly cooled to 15-50°C and, after a standing time of a few minutes up to a number of hours, the crystallized product is filtered off, and the crystallizate is washed with the same solvent and dried.

6. Process for the preparation of chemically and optically highly pure *(R)*- and *(S)*-α-hydroxycarboxylic acids, **characterized in that** it comprises treating the hydrolysis solution obtained by acidic hydrolysis of the *(R)*- and *(S)*-cyanohydrins, prepared by enzyme-catalysed addition of a cyanide group donor to the corresponding aldehydes or ketones, directly with an aromatic hydrocarbon, optionally in combination with a cosolvent, then extracting the mixture at hydrolysis temperature, whereupon after cooling of the organic phase the corresponding chemically and optically highly pure *(R)-* and *(S)*-α-hydroxycarboxylic acids crystallize out.

7. Process according to Claim 6, **characterized in that** chemically and optically highly pure aromatic *(R)-* and *(S)*-α-hydroxycarboxylic acids of the formula Ar-(CH₂)ₙCH(OH)CO₂H in which n is 0 or an integer from 1 to 5 and Ar is an aryl or heteroaryl radical which is unsubstituted or substituted by OH, C₁-C₄-alkyl or -alkoxy, thioalkyl, halogen, optionally substituted phenyl or phenoxy, amino or nitro, are prepared.

8. Process according to Claim 1 or 6, **characterized in that** toluene, xylene, benzene, ethylbenzene, isopropylbenzene or chlorobenzenes are employed as aromatic hydrocarbons.

9. Process according to Claim 1 or 6, **characterized in that** the cosolvent employed is a solvent which increases the solubility of the hydroxycarboxylic acid in the organic phase and which is readily separable by distillation, in an amount from 5 to 50% by volume.

## Revendications

1. Procédé de production d'acides *(R)* et *(S)*-α-hydroxycarboxyliques optiquement et chimiquement hautement purs, **caractérisé en ce que** les acides α-hydroxycarboxyliques *(R)* et *(S)* non purs produits par hydrolyse acide des cyanhydrines *(R)* et *(S)* obtenues par addition par catalyse enzymatique d'un donneur du groupe cyanure à l'aldéhyde ou la cétone correspondants dans un hydrocarbure aromatique, sont cristallisés, le cas échéant en présence d'un co-solvant, et que des acides *(R)* et *(S)* α-hydroxycarboxyliques optiquement hautement purs sont obtenus.

2. Procédé selon la revendication 1, **caractérisé en ce que** les acides *(R)* et *(S)* α-hydroxycarboxyliques non purs sont produits par hydrolyse acide des cyanhydrines *(R)* et *(S)* obtenues par addition par catalyse enzymatique d'un donneur du groupe cyanure à l'aldéhyde aliphatique, aromatique ou hétéro-aromatique éventuellement substitué ou à la cétone correspondants.

3. Procédé selon la revendication 1, **caractérisé en ce que** des acides *(R)* et *(S)* α-hydroxycarboxyliques non purs de formule Ar-(CH₂)ₙCH(OH)CO₂H, dans laquelle n représente 0 ou un nombre entier de 1 à 5 et Ar est un radical aryle ou hétéro-aryle non substitué ou mono- ou poly-substitué par les groupes OH, alkyle ou alcoxy en C₁-C₄, thioalkyle, halogène, éventuellement phényle ou phénoxy, amino ou nitro sont utilisés.

4. Procédé selon la revendication 1, **caractérisé en ce que** de l'acide *(R)*-2-chloromandélique est utilisé.

5. Procédé selon la revendication 1, **caractérisé en ce que** l'acide α-hydroxycarboxylique à purifier est dilué dans le solvant ou le mélange de solvants correspondant en le chauffant, est lentement refroidi à 15-50° C et après un temps de repos de quelques minutes à plusieurs heures, le produit cristallisé est filtré, le cristallisât est lavé fois avec le même solvant et séché.

6. Procédé de production d'acides *(R)* et *(S)*-α-hydrocarboxyliques chimiquement et optiquement hautement purs, **caractérisé en ce que** la solution d'hydrolyse obtenue par hydrolyse acide des cyanhydrines *(R)* et *(S)* produites par addition par catalyse enzymatique d'un donneur du groupe cyanure à l'aldéhyde ou la cétone correspondants, directement avec un hydrocarbure aromatique, le cas échéant en présence d'un co-solvant, est remplacée, ensuite est extraite à une température d'hydrolyse, et après refroidissement de la phase organique, les acides *(R)* et *(S)* α-hydroxycarboxyliques chimiquement et optiquement hautement purs étant cristallisés.

7. Procédé selon la revendication 6, **caractérisé en ce que** les acides *(R)* et *(S)*-α-hydrocarboxyliques chimiquement et optiquement hautement purs, de formule Ar-(CH₂)ₙCH(OH)CO₂H, dans laquelle n représente 0 ou un nombre entier de 1 à 5 et Ar pouvant être un radical aryle ou hétéro-aryle non substitué ou mono- ou poly-substitué par les groupes OH, alkyle ou alcoxy en C₁-C₄, thioalkyle, halogène, éventuellement phényle ou phénoxy, amino ou nitro sont produits.

8. Procédé selon la revendication 1 ou 6, **caractérisé en ce que** l'on utilise comme hydrocarbure aromatique, le toluol, le xylol, le benzol, l'éthylbenzol, l'isopropylbenzol ou le chlorobenzol.

9. Procédé selon la revendication 1 ou 6, **caractérisé en ce qu'**on utilise comme co-solvant, un solvant qui augmente la solubilité de l'acide hydroxycarboxylique dans la phase organique et qui est facilement séparable par distillation, en une quantité de 5 à 50 % en volume.
